Europäisches Patentamt

European Patent Office

Office européen des brevets

(11) Publication number: 0 466 272 A1

# EUROPEAN PATENT APPLICATION

(21) Application number: 91201792.8

(22) Date of filing: 10.07.91

(51) Int. Cl.5: A61B 5/00, A61G 7/05

(30) Priority: 10.07.90 NL 9001571

(43) Date of publication of application:
15.01.92 Bulletin 92/03

(84) Designated Contracting States:
AT BE CH DE DK ES FR GB GR IT LI LU NL SE

(71) Applicant: VIGGO-SPECTRAMED B.V.
Rembrandtlaan 1A
NL-3723 BG Bilthoven(NL)

(72) Inventor: van den Berg, Willem
Schans 26
1261 MK Blaricun(NL)

(74) Representative: Kooy, Leendert Willem et al
OCTROOIBUREAU VRIESENDORP & GAADE
P.O. Box 266
NL-2501 AW The Hague(NL)

(54) Connection device.

(57) A device for providing a connection between a number of sensors (2) and a number of monitor means (3), the sensors (2) being capable of sensing a number of physical parameters of a patient, such as one or more blood pressures, the temperature, the cardiac output and/or the heartbeat. The device comprises a number of lines (4) each of which having one end connected to a sensor (2) and the other end being intended for connection to an input of a junction box (5). The box (5) has an output for connecting one end of a common cable (6) the other end of which being suitable for connection to the monitor means (3).

FIG.1

The invention relates to a device for providing a connection between a number of sensors and a number of monitor means, in which the sensors are capable of sensing a number of physical parameters of a patient, such as one or more blood pressures, the temperature, the cardiac output and/or the heartbeat, comprising a number of lines each of which having one end connected to a sensor and the other end being intended for a connection to a monitor means.

As a result of the fact that ever more accurate sensors as well as processing means and monitor means suited therefor become available, it is common practice nowadays to sense a large number of physical parameters of a patient, not only before, during and/or shortly after an operation, but also on behalf of observation purposes for a longer period inside or outside the hospital. Under such circumstances the patient is connected to usually fixedly positioned medical equipment, particularly monitor means, by means of a large number of lines. These lines may comprise lines for carrying a fluid, electric lines or combinations of fluid lines and electric lines. For instance, a blood pressure sensor for simultaneously measuring blood pressure at a number of places in the body, may comprise a catheter, which, outside the body, leads to a number of fluid lines by means of coupling means, which fluid lines, through a respective transducer, each convert into electric lines which are connected to monitors and possible further fluid lines for e.g. calibration purposes.

However, it is inconvenient that a complex network of lines is thus created, which impedes patient and nursing staff in their freedom of movement. Whenever a patient needs to be moved, e.g. to or from the operating room, the lines have to be disconnected one by one, to be once again connected one by one elsewhere. In order to avoid interchanging lines, which, in some cases, might be fatal to the patient, the lines are provided with indications, e.g., colours, and/or with plugs having mutually different shapes. Nevertheless, comparitively speaking, a lot of time is involved in disconnecting and connecting the lines which is always detrimental to the patient. A further drawback is the fact that relatively long lines are required for bridging the distance between patient and monitor equipment, e.g. two metres, which in the event of fluid lines may result in interfering signals caused by resonance phenomena, which reduce the measuring accuracy.

It is an object of the invention to raise these objections and for this purpose, according to the invention a device of the kind as mentioned in the preamble is provided, characterized in that said device comprises a junction box provided with a number of inputs for the connection of the other ends of the lines thereto, and a common output, being at least electrically connected to the inputs, for connecting the one end of a common cable thereto, the other end of which is suitable for a connection to the monitor means.

By way of illustration, the invention will be elucidated hereinafter with reference to the enclosed drawing, in which:

figure 1 shows a block diagram of an arrangement including the device according to the invention;

figure 2 shows a perspective rear view of an embodiment of the junction box of the device according to the invention; and

figure 3 shows a perspective front view of the junction box according to figure 2.

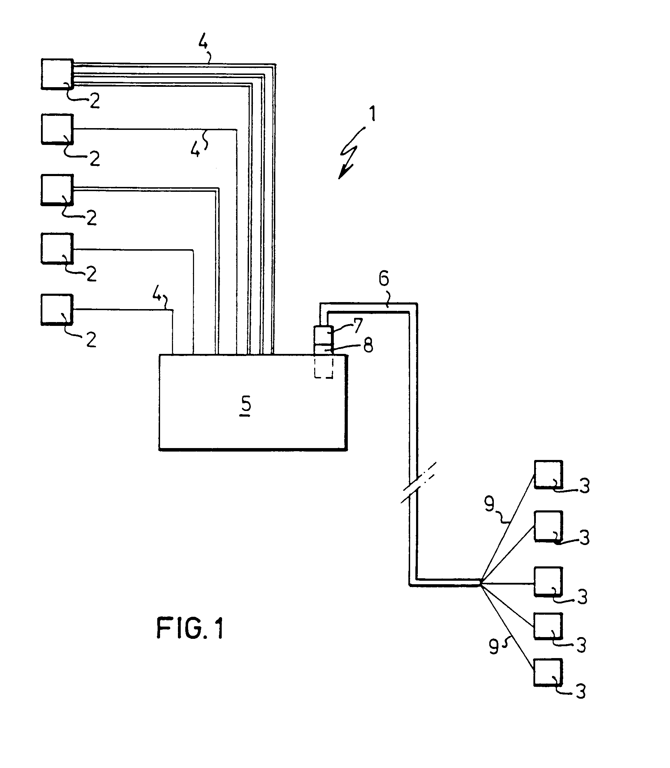

Figure 1 schematically shows a measurement system 1 which comprises a number of sensors 2, five in this example, that are capable of sensing physical parameters of a patient. In this case, particularly those circumstances in which the patient remains connected for a longer period to medical equipment via the sensors 2 are involved. For instance, the sensors 2 are used to measure the temperature at a number of locations in or on the body, the bloodpressure at various locations in the blood circulation system, the cardiac output and the heartbeat. The parameters thus observed by the sensors 2 are, after possible processing, made appropriate for a representation, visually or auditory, by monitor means 3. The monitor means 3 may be of a usual kind, among them monitors with a cathode ray tube, numerical display means, e.g. having liquid crystals, graphic plotters, etc., and, they are fixedly positioned somewhat remote from the patient.

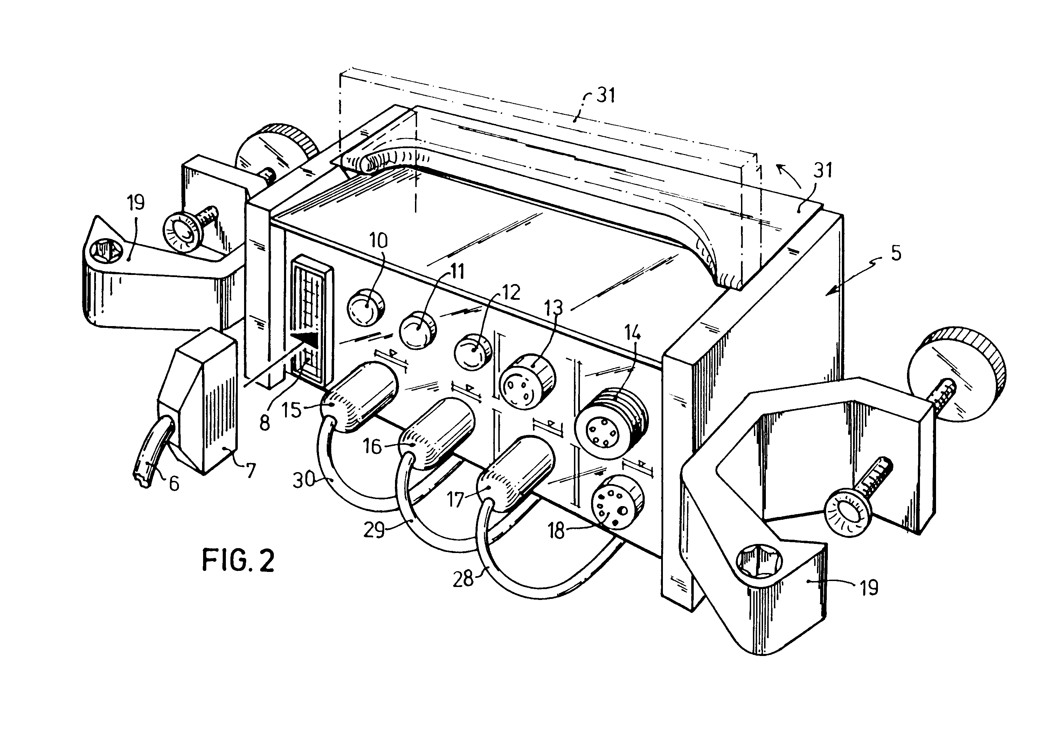

A line 4 is extending from each sensor 2 to a junction box 5. The lines 4 may comprise both electric lines and fluid lines. From the junction box 5 one common cable 6 is extending, which at one end is provided with a multi-plug 7 which can be detachably connected to a matching multi-counterplug 8, see figure 2 also, which forms the output of the junction box 5. At the other end of the cable 6, this cable splits into several lines 9, possibly by the interference of a detachable coupling means, said lines leading to the respective monitor means 3.

The common cable 6 enables, by just one handling, viz. pulling the multi-plug 7 out of, inserting the multi-plug 7 in, the multi-counterplug 8, respectively, to disconnect, connect, respectively, the sensors 2 and with it the patient to the monitor means 3, which as a consequence hardly requires any time. Besides, mutual interchanges of the lines 4 are excluded, for said lines remain connected.

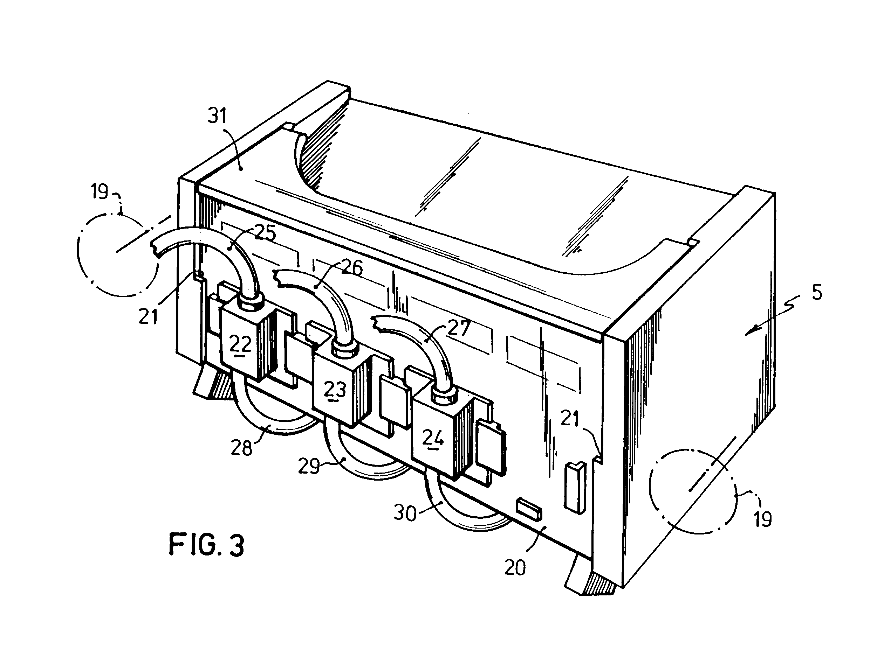

An embodiment of the junction box according to the invention is illustrated in figures 2 and 3. As further illustrated in figure 2, the junction box 5, shows nine inputs 10 to 18 inclusive in this example, i.e. three inputs 10, 11 and 12 for temperature sensors, one input 13 for a cardiac output (c.o.)-sensor, one input 14 for a heartbeat (ecg)-sensor and four inputs 15, 16, 17 and 18 for pressure sensors. As stated before, the output of the junction box is formed by the multi-counterplug 8. The dimensions of the junction box 5 may be 200 x 100 x 100 mm.

The junction box 5 comprises, furthermore, securing means 19 for detachably securing the junction box 5 to a patient supporting device, e.g. a bed or the like. The securing means 19, here in the shape of a grip provided with a securing screw, are also suitable for mounting the junction box 5 to a stand (not shown) positioned close to the patient. Any other form or type of securing means is suitable e.g., incorporating spring members, as long as a reliable securing is provided and a quick mounting or demounting is possible.

Figure 3 shows a front view of the junction box 5. A holder 20, which has been detachably mounted in the junction box 5 via slots 21, carries a number of detachable transducers 22, 23 and 24 for converting the pressure measured in a respective fluid line 25, 26, 27, into an electric signal, which is presented through a short outgoing electric line 28, 29, 30, respectively, said electric lines being connected to the inputs 16, 17, 18 respectively, of the junction box 5 in this case. In connection with requirements of sterility, the holder 20 and transducers 22, 23 and 24 can thus be removed easily to be cleaned or replaced.

When the junction box 5 is mounted to the bed of the patient, the fluid lines can be very short, e.g. 90 cm, without restricting the patient's freedom of movement. From the patient's bed, only the multi-cable 6 is extending to the monitor means 3, thus providing a very conveniently arranged measurement system, wherein the nursing staff will not be impeded by large numbers of connection lines. Moreover, the multi-cable 6 solely carries electric signals. Resonance phenomena and other interfering signals in the fluid lines are prevented as much as possible by the short length thereof.

As the junction box 5 constantly remains at the patient's side even when the patient is disconnected from the monitor means 3 by disconnecting the multi plug 7 and, consequently, the junction box 6 has to be easily transferred or carried along, for instance from the bed to the operating table, the junction box 5 is provided with a handle 31 at its top.

It will be clear to an expert that many modifications of the above-described embodiment of the device according to the invention can be made within the scope of the invention. Thus, the number of inputs of the junction box can be increased or decreased according to one's needs. In the junction box for instance, electric circuits may be incorporated for matching a signal, amplitude or impedance, for example. The inputs of the junction box may, except for the connection of electric lines, also be suitable for a direct connection to fluid lines for instance, in which case proper fluid line connections may be present in the junction box.

## Claims

1. Device for providing a connection between a number of sensors and a number of monitor means, in which the sensors are capable of sensing a number of physical parameters of a patient, such as one or more blood pressures, the temperature, the cardiac output and/or the heartbeat, comprising a number of lines each of which having one end connected to a sensor and the other end being intended for a connection to a monitor means, **characterized in that** a junction box is provided, comprising a number of inputs for connecting the other ends of the lines thereto, and a common output, being at least electrically connected to the inputs, for connecting one end of a common cable thereto, the other end of which being suitable for a connection to the monitor means.

2. Device according to claim 1, **characterized in that** the junction box comprises securing means for securing the junction box to a patient supporting device, such as a bed.

3. Device according to claim 1 or 2, **characterized in that** the junction box comprises a holder which carries one or more transducers, said transducers being electrically connected to one or more inputs of the junction box.

4. Device according to any of claims 1 to 3 inclusive, **characterized in that** the junction box comprises a handle means.

FIG. 1

FIG.2

FIG. 3

EP 0 466 272 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-1 466 807  (ELAG, J. KLEIN)<br>* claims 1-3, 6; figures 1, 4-6 *<br>– – – | 1-4 | A 61 B 5/00<br>A 61 G 7/06 |
| X | CH-A-524 992  (BROWN, BOVERY & CIE)<br>* column 2, line 19 - column 4, line 24 *<br>– – – | 1,3,4 | |
| X | FR-A-1 531 413  (ELECTRONIQUE MARCEL DASSAULT)<br>* page 1, column 1, line 37 - page 3, column 1, line 42; figures *<br>– – – | 1,3 | |
| X | DE-U-8 908 398  (A. DATZ)<br>* claim 1; figure 1 *<br>– – – – – | 1,3 | |

**TECHNICAL FIELDS SEARCHED (Int. Cl.5)**

A 61 B
A 61 G

The present search report has been drawn up for all claims

| Place of search | Date of completion of search | Examiner |
|---|---|---|
| The Hague | 27 August 91 | RIEB K.D. |

CATEGORY OF CITED DOCUMENTS

X : particularly relevant if taken alone
Y : particularly relevant if combined with another
    document of the same catagory
A : technological background
O : non-written disclosure
P : intermediate document
T : theory or principle underlying the invention

E : earlier patent document, but published on, or after
    the filing date
D : document cited in the application
L : document cited for other reasons

& : member of the same patent family, corresponding
    document